(19) Europäisches Patentamt
European Patent Office
Office européen des brevets

(11) **EP 1 600 864 B1**

(12) **EUROPEAN PATENT SPECIFICATION**

(45) Date of publication and mention
of the grant of the patent:
**19.12.2007 Bulletin 2007/51**

(51) Int Cl.:
*G06F 17/50* (2006.01)   *G06F 19/00* (2006.01)

(21) Application number: **04012436.4**

(22) Date of filing: **26.05.2004**

(54) **Modelling tool for chemical processes**

Modellierungs-Werkzeug für chemische Prozesse

Outil de modélisation pour un processus chimique

(84) Designated Contracting States:
**AT BE BG CH CY CZ DE DK EE ES FI FR GB GR HU IE IT LI LU MC NL PL PT RO SE SI SK TR**

(43) Date of publication of application:
**30.11.2005 Bulletin 2005/48**

(73) Proprietor: **Innetics AB**
**583 30 Linköping (SE)**

(72) Inventors:
• **Gunnarsson, Johan**
**586 63 Linköping (SE)**
• **Johansson, Henrik**
**584 31 Linköping (SE)**
• **Jirstrand, Mats**
**431 42 Mölndal (SE)**

(74) Representative: **Edlund, Fabian**
**AWAPATENT AB**
**P.O. Box 11 394**
**404 28 Göteborg (SE)**

(56) References cited:
**WO-A-02/44992**

• **SAURO HERBERT M: "TALIS: Visual, Interactive, Metabolic Simulation" CALIFORNIA INSTITUTE OF TECHNOLOGY, HOMEPAGE HERBERT M SAURON, [Online] 30 October 2001 (2001-10-30), XP002298450 PASADENA, CA, USA Retrieved from the Internet: URL:http: //web.archive.org/web/20011030204 425/http: //www.cds.caltech.edu/~hsauro/Tal is/talis.htm> [retrieved on 2004-09-28] -& SAURO HERBERT M: "Talis setup program (Printout: "TALIS.TXT")" CALIFORNIA INSTITUTE OF TECHNOLOGY, HOMEPAGE HERBERT M SAURON, [Online] 17 November 2001 (2001-11-17), XP002298401 PASADENA, CA, USA Retrieved from the Internet: URL:http://web.archive.org/web/20011117033 120/www.cds.caltech.edu/~hsauro/Talis/tset up.exe> [retrieved on 2004-09-28] -& EXAMINER: "Talis Screenshots" SCREEN PRINTOUT DOCUMENT, 28 September 2004 (2004-09-28), XP002298718 EPO MUNICH**
• **ZHANG BAOXIN: "PATHWAY EDITOR TOOL - USER MANUAL" HOMEPAGE CENTER FOR COMPUTATIONAL GENOMICS, CASE WESTERN RESERVER UNIV., [Online] 2 December 2003 (2003-12-02), XP002298451 CLEVELAND, OH, USA Retrieved from the Internet: URL:http: //web.archive.org/web/20031202170 515/http:// nashua.cwru.edu/PathwaysRelease 1/PathwayEditorUserManual.V1.R7.pdf> [retrieved on 2004-09-28]**

**Description**

<u>Technical field</u>

The present invention relates to modelling tools for biochemical processes, and in particular the graphical modelling present in such tools.

<u>Technical background</u>

Biochemical reaction networks or pathways have been utilized for decades in biology and medicine to graphically describe cellular processes. The aim of these pictures ranges from sketches of qualitative findings to very accurate descriptions of molecular mechanisms. Hence, the effort of transforming these graphical formalisms to mathematical models that can be validated by experimental data varies substantially. The recent advances in experimental techniques and the ever increasing amount of data have triggered a shift of focus in many biological fields from an entity oriented view to a systems approach, i.e., from studying the parts in isolation to the study of how they interact and their functional roles. This scientific area is known as systems biology. Mathematical models are indispensable tools in this work and there is a need for software that supports mathematical modelling, computational analysis, and documentation but is still easy to use.

Modelling tools are known in the art that provide a biological notion that is suitable to graphically describe pathways. A typical modelling tool achieves this by providing a standard library of object templates which are adapted to graphically describe biological objects such as for example molecules, reaction arrows, control arrows and membranes. At the same time, each object template represents a mathematical model of the corresponding molecule, reaction arrow, control arrow or membrane. The tool is thus capable of determining a mathematical model based on the graphical representation created by the user.

For a biologist building pathway models, with such a tool the knowledge and perspective of the pathway model will evolve over time. Many different approaches will be evaluated in the model since the modelling tool lets the user perform numerical studies of how the model can be changed in order to fulfill the goal that the researcher strives at.

For a new biochemical interaction in a pathway the knowledge will typically be very limited in the first place. This means that the modeler will start by taking very simple forms of interaction in the first stage, and then when more knowledge and experiences are collected the details will be more elaborate, leading to a more complex description of the interaction where more and more interacting relationships might be involved. To support the evolution of the interaction knowledge it is desirable for the tool user to be able to improve the interaction models in steps. However, in the modelling tools mentioned above, where the graphical language has been derived from a mathematical view point, adjustment of the model may be problematic.

In a conventional model tool an interaction object is an instance of a ready made object template. To simply extend the interaction, such as add the influence of an additional molecule, to an existing interaction object, is not possible if the interaction object in question is not adapted to handle another molecule. Instead the user has to find another interaction object template that happens to have support for another molecule. Consequently, the user then has to disassemble the old pathway, replace the old interaction object with this new interaction object, reassemble the pathway, and assign important model data to the new interaction object. In order to achieve a functioning model, the relationships between other interaction objects in the pathway and the substituted interaction object also have to be reconfigured. For an extensive pathway the reconfiguration process is very time-consuming and it also has to be performed repeatedly, for each improvement of the pathway model.

Similarly, the opposite change of a pathway, i.e. removing the influence of a molecule on a certain interaction, produces the corresponding problem of replacing an interaction object by disassembling the pathway, removing the old interaction object, inserting a new interaction object, reassembling the pathway, assigning new data to the new interaction objects and reconfiguring the relationships between the interaction objects in order to get a functioning model.

<u>Summary of the invention</u>

An object of the invention is to alleviate at least some of the drawbacks mentioned above facilitating amendment of an interaction complex in a computer displayed graphical model of a biochemical process.

According to a first aspect of the invention, this and other objects, which will appear from the following description, have now been achieved by a method of amending an interaction complex in a computer displayed graphical model of a biochemical process, the graphical model corresponding to a mathematical representation in which said interaction complex is associated with a flow rate between at least two entities in the process. The method comprises receiving input from a user indicating an amendment of the interaction complex by addition of an interaction object to the interaction complex, the interaction object being associated with an additional entity variable and having at least one terminal point, graphically connecting the terminal point to the interaction complex, and displaying an updated interaction complex, and, in the mathematical representation, associating the updated interaction complex with one single flow rate, dependent on the at least two entity variables and the additional entity variable.

According to a second aspect of the invention, the objects of the invention have been achieved, in correspondence with to the above mentioned method, where the amendment is removing an interaction object from the interaction complex.

In both cases it is thus a matter of updating the interaction complex graphically and then automatically updating the mathematical representation to account for the entity variables present in the interaction complex after the amendment.

The invention is based on the understanding that an amendment of an existing interaction complex indicated by a user can be used both to display an update of a graphical model of a biochemical process and to associate the interaction complex with a new mathematical property.

In particular, the invention is based on the understanding that such an amendment indicated by a user can be used to associate the interaction complex with a flowrate which is dependent on the entity variables connected to the interaction complex.

Preferably, when connecting an interaction object, the method also comprises the step of removing any association to an entity variable from the terminal point. This simplifies the reconfiguration of an interaction complex since the user not has to remove such an association manually. Instead, the connecting of the terminal point to an interaction complex automatically removes any association that terminal point previously has had to an entity variable.

In correspondence it is also preferred that, in the case of removing an interaction object from an interaction complex, an association is created between the terminal point and an entity variable. In this case the disconnecting of the interaction object is the action that automatically creates a new association.

Preferably, the connected interaction object is provided with a reference to the object it has been connected to, creating a logical connection between the objects thereby ensuring coordinated treatment of the objects. By comprising this step the method enables the creation of a tree hierarchy between objects in an interaction complex. Such a relation hierarchy is useful when the user handles composite interaction objects, for example when adding several interconnected interaction objects at the same time.

In correspondence it is also preferred that, in the case that an indication of a removing of an interaction object from an interaction complex is received, any reference from the disconnected interaction object to the object it was previously connected to is removed, thereby removing any logical connection between the objects.

The logical connection can enable control of the connected interaction object by controlling of the object it has been connected to. By enabling this control a user indicated amendment of an object having a higher position in the tree hierarchy can be used to cause corresponding amendments of objects having an inferior position in the tree hierarchy. In this way similar amendments, both graphical and mathematical, can be ensured for several objects at the same time, in a short time. A simple example is the ability to move several objects at the same time, by drag and dropping the object they refer to.

The mathematical representation typically comprises a set of time derivatives of entities involved in the biochemical process, each time derivative being defined as a sum of contributions, where each contribution is proportional to a flow rate. By including this type of time derivatives in the mathematical representation it is possible to achieve a mathematical expression for the flow rate of the interaction complex based on contributions from each entity variable in the interaction complex. This correlation between the flow rate of the interaction complex and separate entities facilitates the analysis of an amendment of an interaction complex; the user can easily distinguish consequences of, for example an adding of an interaction object with an entity variable to an interaction complex, on the flow rate of the interaction complex.

Preferably, the contribution from a specific interaction complex to a time derivative of an entity variable is proportional to a stoichiometric coefficient defined for the entity variable in this interaction complex. This ensures that the sum of contributions corresponds to a biochemical reaction formula having the right proportions between the participating entities.

A method according to the invention typically also comprises the step of automatically updating the set of time derivatives in accordance with the updated interaction complex, in order to achieve a correct relative consumption and production respectively of each substrate and product entity. This automatic achievement secures that the user is provided with the correct information of the updated relative consumptions and productions without having to perform any other reconfigurations than the above mentioned amendment itself.

The above method can be implemented in a system for amendment of an interaction complex.

Brief description of the drawings

The invention will now be described in more detail with reference to the accompanying schematic drawings which show preferred embodiments of the invention and in which:

fig. 1 shows a schematic overview of a system according to the invention;

fig. 2 shows an example of an interaction complex according to the invention;

fig. 3A and 3B show how an interaction object is added to the interaction complex in fig 2;

fig. 3C shows how a control object is removed from the interaction complex in fig 2;

fig. 4 is a flow chart illustrating an embodiment of a method according to the invention.

Detailed description of a preferred embodiment

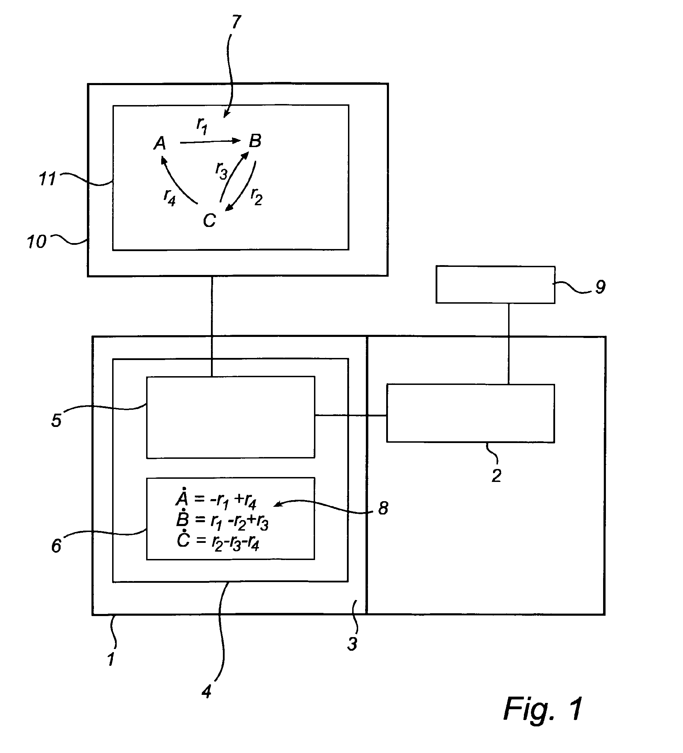

With initial reference to fig. 1, there is shown an environment in which the method according to the invention can be utilized. A computer 1 comprises a processor unit 2 and a memory area 3. A modelling tool 4 is installed on the memory area 3 and runs on the computer 1. The modelling tool 4 comprises a graphical module 5, arranged to display a graphical model 7 of a biological pathway on the working area 11 of a display unit 10, and a computational module 6, arranged to handle a mathematical representation 8 of the model 7.

The model 7 includes a number of entities A, B, C and a number of flow rates $r_1$, $r_2$, $r_3$ and $r_4$. The mathematical representation 8 is essentially a set of differential equations where the time derivative of an entity is set equal to the sum of flow rates connected to this entity.

A user can interact with the modelling tool 4 on the memory area 3, giving instructions to the modelling tool through an input device 9, such as a keyboard or a mouse. The modelling tool presents the results of the user's actions on the display unit 10, where operations corresponding to said actions are performed. The operations may include indicating an amendment of an interaction complex in the model 7 by addition of an interaction object to the interaction complex. It should be noted that the model 7 may consist of several interconnected interaction complexes.

The term interaction object refers to a kind of modelling object that serves to model an interaction of an entity, such as molecules or atoms, on another entity or a process. Correspondingly the term interaction complex refers to one or several such interaction objects representing a flow between at least two entities in the pathway model 7. In fig 1, the flow rates $r_1$ - $r_4$ are each associated with a separate interaction complex. The interaction objects of an interaction complex can, according to the invention, be connected to each other by the user in order to achieve a customized model object that is suitable for representing a complex interaction between several entities. In the following description and figures, the object being moved by the user is assumed to be a single interaction object, while the interaction complex is assumed to comprise several interaction objects. However, this is only an example, and as will be noted below, the situation may well be different, i.e. the modelling object moved by the user may comprise several interaction objects, and the stationary modelling object may consist of one single interaction object.

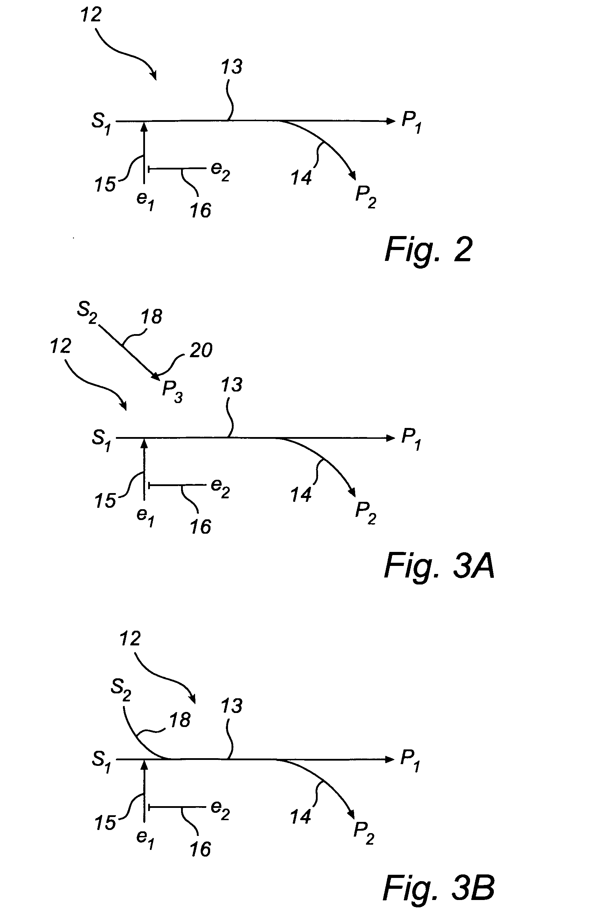

Fig. 2 illustrates an interaction complex 12 representing a chemical reaction from a substrate entity to the two product entities, having a flow rate r. The entities are represented by variables $s_1$, $p_1$ and $p_2$. The flow rate is influenced by the modulator entities $e_1$ and $e_2$. The interaction complex 12 constitutes one part of the pathway 7, and the flow rate r is thus one of the factors influencing the system of differential equations in the mathematical representation. It should be noted that the modulators e1 and e2 only influence the reaction without being consumed nor produced. In other words, the flow rate of the complex 12 will only influence the time derivatives of the entities $s_1$, $p_1$ and $p_2$.

The complex 12 can be regarded as being composed by a multitude of interaction objects 13, 14, 15 and 16.

Two of the objects, 13 and 14, are reaction objects, each by itself representing a chemical reaction with a flow rate from a substrate to a product. As they now are joined together, they will share the same flow rate (the reaction cannot take place with a different rate in one part of the reaction than others). Each object 13, 14, has two terminal points. Some of these are "free", and associated with an entity variable ($s_1$, $p_1$, $p_2$). Others, like the begin point of object 14, are not free, and are not connected to an entity variable.

Interaction objects 15 and 16 are control objects, and have slightly different function than objects 13 and 14, as they do not represent a flow but only an influence on the flow rate of an interaction complex. Consequently, only one of their terminal points can be free (and associated with an entity variable), while the other (the end point) has to be connected to another object in order for the control object to have a purpose in the mathematical representation. Graphically, the objects 13 and 14 are not connected integrally with the object they are attached to. In the illustrated example this is illustrated by having different types of terminal points, which indicates representations of different biomechanical functions. The interaction object 15 (ending in an arrow head) represents a promotion which initiates or accelerates the biochemical reaction it is connected to, so that the entity variable $e_1$ here acts as a promotor in the flow rate of the reaction from $s_1$ to $p_1$ and $p_2$. Similarly, the interaction object 16 (ending in a T-shape) represents an inhibition which terminates or decelerates the reaction it is connected to, so that the entity variable $e_2$ here acts as an inhibitor in the influence from $e_1$. The entities $e_1$ and $e_2$, generally referred to as modulators, can be for example enzymes, but can also be e.g. ions or molecules.

Each individual object preferably refers to the object to which it is attached. For example, objects 14 and 15 refer to object 13, and object 16 refers to object 15. An interaction complex can therefore be regarded as a tree structure, where attached objects are branches, and where the original object, i.e. the object that does not refer to any other object (in the illustrated case object 13) can be referred to as the "root" object. It should be noted that a control object, which by definition must be connected to another object in order to have a function, never can be a root object (at least not in an interaction complex playing an active role in the mathematical representation).

The reference can be a "two-way" reference, meaning that also the referred object (e.g. 13) will be aware of the referring object (e.g. 14). This enables the modelling tool to establish all dependencies in the tree structure from any given starting point.

The dependency structure enables the entire interaction complex 12 to graphically behave like one object. When a user drags and drops a part of the complex (e.g. with the mouse pointer), all objects referring to that object will move together. In the present case, this means that moving object 13 will move the entire complex 12. Moving object 14 will, on the other hand, disconnect it from object 13, as will be described below.

Preferably, the interaction object also comprises a set of parameters representing the stoichiometric coefficients of the different entities in the chemical reaction underlying the complex. In the illustrated example, a chemical reaction formula corresponding to the interaction complex 12 could look like:

$$2s_1 + e_1 + e_2 \Rightarrow 3p_1 + p_2 + e_1 + e_2,$$

in which case the coefficient of $s_1$ is 2, the coefficient of $p_1$ is 3, and all other coefficients are 1. From the chemical reaction formula it can be verified that the enzymes $e_1$ and $e_2$ only represent a catalytic influence in the reaction and are not consumed nor produced.

The flow rate of the reaction above can be expressed as:

$$r = r(s_1, e_1, e_2, p_1, p_2),$$

indicating that it is a function of all entities connected to the interaction complex 12. As was mentioned, the flow rate must be the same in the entire interaction complex 12, and influences the time derivatives of the substrate $s_1$ and products $p_1$, $p_2$ in the mathematical representation.

For the interaction complex 12 with the above chemical reaction formula and flow rate r, the contributions to the time derivatives in the mathematical representation will be:

$$\dot{s}_1 = -2r(s_1, p_1, p_2, e_1, e_2)$$

$$\dot{p}_1 = +3r(s_1, p_1, p_2, e_1, e_2)$$

$$\dot{p}_2 = +r(s_1, p_1, p_2, e_1, e_2).$$

As can be seen, the time derivate contribution to the substrate variable $s_1$ is negative, indicating consumption, while the time derivatives for the product variables $p_1$ and $p_2$ are positive indicating production. It should also be noted that the stoichiometric coefficients input by the user influence the time derivative contributions. The time derivative contribution thus relates not only to the flow rate of the interaction complex, but also exhibits a positive or negative direction of flow and a proportional coefficient.

As explained, expressions above will form one contribution to the set of differential equations in the mathematical representation in the software unit 6. However, the mathematical representation describes the relationship between several interaction complexes influencing each other (see fig 1). In other words, the time derivative for an entity variable is influenced not only by the flow rate of one interaction complex, but by the flow rates of all interaction complexes involving the entity as either a substrate or product.

It should be noted that the interaction complex 12 from the beginning only is an empty model object. The exact relationships between the flow rate r and the entities $s_1$, $p_1$, $p_2$, $e_1$ and $e_2$ have to be entered into the model by the user, by defining a formula, specific parameters and their values. Another set of parameters entered by the user are the stoichiometric coefficients of the entities in the complex.

Fig. 3A and 3B show how an interaction object 18 is connected to an interaction complex 12 in a pathway 7. As can be seen, this interaction object 18 is a reaction object representing a chemical reaction from a substrate $s_2$ to a

product $p_3$.

An object can be added to an existing complex by one of its terminal points (begin or end point). If the interaction object to be added itself is a complex, i.e. consists of several objects, it is only the terminal points of the root object that can be connected to another complex. The terminal points of the interaction object can be connected anywhere on the existing interaction complex. A terminal point that has been connected to another object will no longer be "free", and will thus not be associated with any entity variable. The model keeps track of the status of each terminal point in order to determine which are free and which are not.

In the present case, the terminal point 20 of interaction object 18 is connected to the mid section of the reaction object 13 of the interaction complex 12 (fig 3B). The status of terminal point 20 is changed to "unfree". As explained above, the added object 18 will refer to the object 13 to which it has been added, and will move together with it.

The adding of the interaction object 18 also causes an adjustment of the mathematical representation 6, taking the added substrate variable $s_2$ into consideration. First, the flow rate of the reaction object 18 is removed, while the flow rate of the updated interaction complex 12 is automatically redefined to allow for influence of entity variable $s_2$, i.e.:

$$r = r(s_1, s_2, e_1, e_2, p_1, p_2).$$

The exact relationship typically is defined by the user, who now can involve the variable $s_2$ in the equation. Suitable stoichiometric coefficents can also be added or adjusted by the user. Of course, the system may be combined with some kind of database and/or AI system, further assisting the user with defining the correct relationships and coefficients for the modeled reactions. The graphical interface may then provide a tool for creating an entire simulation model, without requiring additional input from the user. This, however, does not form part of the present invention, and will not be further described.

The set of time derivatives in the representation 6 are then updated in accordance with the updated chemical reaction formula and the updated flow rate and now also comprises the time derivative indicating the consumption of the substrate entity variable $s_2$ (if this was not already part of the pathway 7).

It should be noted that the adding of a new interaction object 18 and a new entity variable $s_2$ to a pathway 7 corresponds to the creation of a totally new chemical reaction which not necessarily may have much resemblance with the original biochemical process. For instance the updated pathway 7 may have new product entities replacing the original ones, and new stoichiometric coefficients may replace the old ones for any entity variable in the pathway. This information has to be completed by the user in order to achieve a proper representation of the biochemical process.

If the added object had been a control object, the procedure above would have been the same, with the exception that the control object does not have any flow rate.

Instead of adding object 18 to complex 12, complex 12 could have been added to object 18. As mentioned above, it would then have to be one of the terminal points of root object 13 that were connected to object 18. In this case, object 18 would become the new root object, the flow rate of the original complex 12 would be removed, and the flow rate of the updated complex would be equal to the flow rate of object 18.

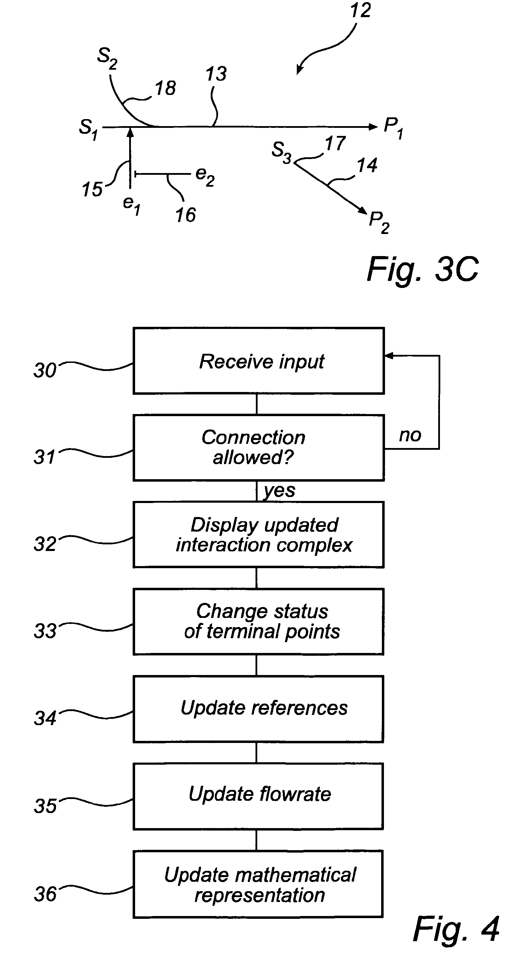

Further, fig. 3C illustrates an amendment of the interaction complex 12 in fig. 3B by removing an interaction object, namely the reaction object 14, from the interaction complex 12.

When the user drags and drops the object 14, this does not cause the entire complex to move, as it is not the root object. Instead, the object 14 is disconnected from the complex 12, and the status of the disconnected terminal point 17 is changed to "free", and is again associated with an entity variable $s_3$.

In the illustrated case, the object 14 does not have any objects (reaction nor control) referring to it. If further objects had been connected to object 14 as referring objects (further down in the tree structure), such objects would have been moved together with object 14, to form a new composite object with object 14 as the root object. In any case, the disconnected object will inherit a set of common parameters (including the flow rate) from the root object 13 of the original complex 12. This ensures the consistency of the model even if a user disconnects a part of a complex and then connects it again.

Note that the flow rate of the disconnected object most probably depends on variables (entities) no longer connected to this complex. If the user attempts to simulate the model at this stage, an error message is generated, and the user is encouraged to amend the expression for this flow rate. In principle, the opposite applies when an object and an additional entity variable is connected to a complex, i.e. that the flow rate initially does NOT depend on an existing entity variable. Even though this does not create a mathematical inconsistency (and hence does not require an error message), it does represent some kind of logical inconsistency, as the user has indicated the influence of an entity variable without any mathematical correspondence. Therefore, it may be desired to present some type of control message in this case.

When an object (or group of object) is disconnected from the complex 12, the definition of the flow rate is updated, illustrating the removed dependence on $p_2$:

$$r = r(s_1, s_2, e_1, e_2, p_1).$$

The exact relationship is again amended by the user, and suitable stoichiometric coefficients are added or adjusted by the user.

The time derivatives in the set are then updated, to account for the disconnection of product $p_2$ from this interaction complex, and for any changes to the stoichiometric coefficients.

It should be noted that the process described above would have been analogous if a control object (e.g. control object 16) would have been removed. In this case, however, the disconnected object would not inherit any flow rate, as it would contain only a control object. In fact, this disconnected control object will completely cease to influence the mathematical representation of the pathway.

Fig. 4 illustrates schematically a flowchart of the method described above. In step 30, the modelling tool 4 receives an input from a user indicating an amendment of an interaction complex in the biochemical pathway 7, for example an addition or deletion of an interaction object as described above. This input is typically a "drag and drop" action performed using the pointing device 9.

In step 31 it is determined whether an intended connection is allowed or not. As described above, this requires that the terminal point of the interaction object to be connected must be a terminal point of the root object .

If the connection is not allowed, the workflow returns to step 30 and awaits further input. If the indicated connection is allowed, or if the amendment is a disconnection, program control continues to step 33. Here, the modelling tool reacts to the input by graphically displaying the amendment, i.e. connecting a terminal point of the interaction object to the interaction complex, or removing an interaction object from the interaction complex, and displays an updated interaction complex 12 on the working area 11 of the display device 10.

In step 33 the status of the terminal point of the interaction object that was connected or disconnected to/from the interaction complex is updated. Any disconnected terminal point 17 (including a disconnected terminal point of the remaining interaction complex 12) will accordingly receive status "free", while any connected terminal 20 point will receive status "unfree" (in fact, as long as the object 18 is connected to the complex 12, the terminal point 20 does not act as a terminal point at all).

In step 34, the modelling tool updates the dependencies between objects in a complex. Thus, a connected object 18 is given a reference pointer to the object 13 it is connected to. Similarly, a disconnected object (or complex) is cleared from references to objects no longer part of the same complex, and a new root object is determined (as mentioned above, the new root object will typically be the object that the user dragged and dropped).

Further, in step 35, the updated interaction complex is associated with one single flow rate which is dependent on the entities now connected to the complex. In the case of an addition, this means an extended set of entities, while in the case of a disconnection, this means a reduced set of entities. In the case of a connection, this also involves overwriting a flow rate previously defined in an added reaction object 18 with the flow rate defined in the root object of the interaction complex 12.

Then, in step 36, the expressions for the time derivatives in the mathematical representation of the pathway (possibly also for entities connected to other parts of the pathway 7 than the amended interaction complex 12) are updated with any new contributions resulting from the amended interaction complex. Typically, the number of time derivatives (number of entities) will change each time a reaction object has been added or removed, but not when a control object has been added or removed.

It should be noted that the above description only serves as an example of embodiments of the invention defined by the appended claims. In its most general form, the invention is related to providing a more dynamic modelling tool, allowing the user to amend an interaction object "on the fly", without requiring a tedious template replacement.

## Claims

1. Method of amending an interaction complex (12) in a computer displayed graphical model (7) of a biochemical process, said graphical model corresponding to a mathematical representation (6) in which said interaction complex (12) is associated with a flow rate (r) between at least two entity variables ($s_1$, $p_2$) in the process,

   **characterized by**

   receiving input from a user indicating an amendment of the interaction complex by addition of an interaction object (18) to the interaction complex,

   said interaction object having a first terminal point (17) being associated with an additional entity variable ($s_2$) and

a second terminal point (20), said interaction object representing a chemical reaction of said additional entity variable $(s_2)$ or an influence of said additional entity variable $(s_2)$ on said flow rate (r),
graphically connecting one of said first and second terminal points (17, 20) to the interaction complex (12), and displaying an updated interaction complex, and
in the mathematical representation (6), associating the updated interaction complex (12) with one single flow rate (r), dependent on said at least two entity variables $(s_1, p_1)$ and said additional entity variable $(s_2)$.

2. Method of amending an interaction complex according to claim 1, further comprising removing any association from the connected terminal point (20) to an entity variable.

3. Method of amending an interaction complex according to claim 1 or 2, **characterized by** providing the connected interaction object (18) with a reference to the object (13) it has been connected to, creating a logical connection between the objects thereby ensuring coordinated treatment of the objects.

4. Method of amending an interaction complex (12) in a computer displayed graphical model (7) of a biochemical process, said graphical model corresponding to a mathematical representation (6) in which said interaction complex (12) is associated with a flow rate (r) between at least two entity variables $(s_1, p_1)$ in the process,
**characterized by**
receiving input from a user indicating an amendment of the interaction complex by removing of an interaction object (14) from the interaction complex,
said interaction object having a first terminal point (17) being associated with an entity variable $(s_2)$ and a second terminal point (20), said interaction object representing a chemical reaction of said additional entity variable $(s_2)$ or an influence of said additional entity variable $(s_2)$ on said flow rate (r),
graphically disconnecting one of said first and second terminal points (17, 20) from the interaction complex (12), and displaying an updated interaction complex, and
in the mathematical representation (6), associating the updated interaction complex (12) with one single flow rate (r), dependent on the remaining entity variables $(s_1, p_1)$.

5. Method of amending an interaction complex according to claim 4, further comprising creating an association from the disconnected terminal point (17) to an entity variable.

6. Method of amending an interaction complex according to claim 4 or 5, **characterized by** removing a reference from the disconnected interaction object (18) to the object (13) it has been connected to, removing any logical connection between the objects.

7. Method of amending an interaction complex according to claim 3 or 6, wherein said logical connection enables control of the connected interaction object (18) by controlling of the reference object (13) it has been connected to.

8. Method of amending an interaction complex according to one of the preceding claims, **characterized in that** said mathematical representation comprises a set of time derivatives of entities involved in the biochemical process, each time derivative being defined as a sum of contributions, where each contribution is proportional to a flow rate (r).

9. Method of amending an interaction complex according to claim 8, **characterized by** that the contribution from a specific interaction complex to a time derivative of an entity variable is proportional to a stoichiometric coefficient defined for the entity variable in this interaction complex.

10. Method of amending an interaction complex according to claim 8 or 9, **characterized by** automatically updating the set of time derivatives in accordance with the updated interaction complex, in order to achieve a correct relative consumption and production respectively of each substrate and product entity.

11. System for amending an interaction complex (12) in a computer displayed graphical model (7) of a biochemical process, said graphical model corresponding to a mathematical representation (6) in which said interaction complex (12) is associated with a flow rate (r) between at least two entity variables $(s_1, p_2)$ in the process, **characterized by**

- means for receiving input from a user indicating an amendment of the interaction complex by addition of an interaction object (18) to the interaction complex,
said interaction object having a first terminal point (17) being associated with an additional entity variable $(s_2)$ and a second terminal point (20), said interaction object representing a chemical reaction of said additional

entity variable ($s_2$) or an influence of said additional entity variable ($s_2$) on said flow rate (r),
- means for graphically connecting one of said first and second terminal points (17, 20) to the interaction complex (12), and displaying an updated interaction complex, and
- means for, in the mathematical representation (6), associating the updated interaction complex (12) with one single flow rate (r), dependent on said at least two entity variables ($s_1$, $p_1$) and said additional entity variable ($s_2$).

**12.** System for amending an interaction complex (12) in a computer displayed graphical model (7) of a biochemical process, said graphical model corresponding to a mathematical representation (6) in which said interaction complex (12) is associated with a flow rate (r) between at least two entities ($s_1$, $p_1$) in the process, **characterized by**

- means for receiving input from a user indicating an amendment of the interaction complex by removing of an interaction object (14) from the interaction complex,
said interaction object having a first terminal point (17) being associated with an entity variable ($S_2$) and a second terminal point (20), said interaction object representing a chemical reaction of said additional entity variable ($s_2$) or an influence of said additional entity variable ($s_2$) on said flow rate (r),
- means for graphically disconnecting one of said first and second terminal points (17, 20) from the interaction complex (12), and displaying an updated interaction complex, and
- means for, in the mathematical representation (6), associating the updated interaction complex (12) with one single flow rate (r), dependent on the remaining connected at least two entities ($s_1$, $p_1$).

**Patentansprüche**

**1.** Verfahren zum Ändern eines Wechselwirkungskomplexes (12) in einem auf einem Rechner angezeigten grafischen Modell (7) eines biochemischen Prozesses, das einer mathematischen Darstellung (6) entspricht, bei der der Wechselwirkungskomplex (12) mit einer Durchflussgeschwindigkeit (r) zwischen mindestens zwei Entitätsvariablen ($s_1$, $p_2$) in dem Prozess assoziiert wird,
**gekennzeichnet durch**
Erhalten einer Eingabe von einem Nutzer, die eine Änderung des Wechselwirkungskomplexes **durch** Hinzufügen eines Wechselwirkungsobjektes (18) zu dem Wechselwirkungskomplex angibt, wobei das Wechselwirkungsobjekt einen ersten Endpunkt (17), der mit einer zusätzlichen Entitätsvariablen ($s_2$) assoziiert ist, und einen zweiten Endpunkt (20) hat und das Wechselwirkungsobjekt eine chemische Reaktion der zusätzlichen Entitätsvariablen ($s_2$) oder einen Einfluss der zusätzlichen Entitätsvariablen ($S_2$) auf die Durchflussgeschwindigkeit (r) darstellt,
grafisches Verbinden des ersten oder des zweiten Endpunkts (17, 20) mit dem Wechselwirkungskomplex (12) und Anzeigen des aktualisierten 'Wechselwirkungskomplexes und
Assoziieren, in der mathematischen Darstellung (6), des aktualisierten Wechselwirkungskomplexes (12) mit einer einzelnen Durchflussgeschwindigkeit (r) in Abhängigkeit von den mindestens zwei Entitätsvariablen ($s_1$, $p_1$) und der zusätzlichen Entitätsvariablen ($s_2$).

**2.** Verfahren zum Ändern eines Wechselwirkungskomplexes nach Anspruch 1, das weiterhin das Entfernen einer Assoziation von dem verbundenen Endpunkt (20) zu einer Entitätsvariablen umfasst.

**3.** Verfahren zum Ändern eines Wechselwirkungskomplexes nach Anspruch 1 oder 2, **dadurch gekennzeichnet, dass** das verbundene Wechselwirkungsobjekt (18) mit einer Referenz zu dem Objekt (13), mit dem es verbunden worden ist, versehen wird, wodurch eine logische Verbindung zwischen den Objekten hergestellt wird, sodass eine koordinierte Verarbeitung der Objekte gewährleistet wird.

**4.** Verfahren zum Ändern eines Wechselwirkungskomplexes (12) in einem auf einem Rechner angezeigten grafischen Modell (7) eines biochemischen Prozesses, das
einer mathematischen Darstellung (6) entspricht, bei der der Wechselwirkungskomplex (12) mit einer Durchflussgeschwindigkeit (r) zwischen mindestens zwei Entitätsvariablen ($s_1$, p1) in dem Prozess assoziiert wird,
**gekennzeichnet durch**
Erhalten einer Eingabe von einem Nutzer, die eine Änderung des Wechselwirkungskomplexes **durch** Entfernen eines Wechselwirkungsobjektes (14) aus dem Wechselwirkungskomplex angibt, wobei das Wechselwirkungsobjekt einen ersten Endpunkt (17), der mit einer Entitätsvariablen ($s_2$) assoziiert ist, und einen zweiten Endpunkt (20) hat und das Wechselwirkungsobjekt eine chemische Reaktion der zusätzlichen Entitätsvariablen ($s_2$) oder einen Einfluss der zusätzlichen Entitätsvariablen ($s_2$) auf die Durchflussgeschwindigkeit (r) darstellt,
grafisches Trennen des ersten oder des zweiten Endpunkts (17, 20) von dem Wechselwirkungskomplex (12) und Anzeigen des aktualisierten Wechselwirkungskomplexes und

Assoziieren, in der mathematischen Darstellung (6), des aktualisierten Wechselwirkungskomplexes (12) mit einer einzelnen Durchflussgeschwindigkeit (r) in Abhängigkeit von den übrigen Entitätsvariablen ($s_1$, $p_1$).

5.  Verfahren zum Ändern eines Wechselwirkungskomplexes nach Anspruch 4, das weiterhin das Herstellen einer Assoziation von dem getrennten Endpunkt (17) zu einer Entitätsvariablen umfasst.

6.  Verfahren zum Ändern eines Wechselwirkungskomplexes nach Anspruch 4 oder 5, **dadurch gekennzeichnet, dass** eine Referenz von dem getrennten Wechselwirkungsobjekt (18) zu dem Objekt (13), mit dem es verbunden worden ist, entfernt wird, wodurch eine logische Verbindung zwischen den Objekten beseitigt wird.

7.  Verfahren zum Ändern eines Wechselwirkungskomplexes nach Anspruch 3 oder 6, **dadurch gekennzeichnet, dass** die logische Verbindung eine Steuerung des verbundenen Wechselwirkungsobjektes (18) **dadurch** ermöglicht, dass das Referenzobjekt (13), mit dem es verbunden worden ist, gesteuert wird.

8.  Verfahren zum Ändern eines Wechselwirkungskomplexes nach einem der vorhergehenden Ansprüche, **dadurch gekennzeichnet, dass** die mathematische Darstellung eine Gruppe von Zeitableitungen von an dem biochemischen Prozess beteiligten Entitäten aufweist, wobei jede Zeitableitung als Summe von Kontributionen definiert ist und jede Kontribution proportional zu einer Durchflussgeschwindigkeit (r) ist.

9.  Verfahren zum Ändern eines Wechselwirkungskomplexes nach Anspruch 8, **dadurch gekennzeichnet, dass** die Kontribution von einem speziellen Wechselwirkungskomplex zu einer Zeitableitung einer Entitätsvariablen proportional zu einem stöchiometrischen Koeffizienten ist, der für die Entitätsvariable in diesem Wechselwirkungskomplex definiert ist.

10. Verfahren zum Ändern eines Wechselwirkungskomplexes nach Anspruch 8 oder 9, **dadurch gekennzeichnet, dass** die Gruppe von Zeitableitungen entsprechend dem aktualisierten Wechselwirkungskomplex automatisch aktualisiert wird, um einen genauen relativen Verbrauch bzw. eine genaue relative Erzeugung jeder Substrat- und Produkt-Entität zu erhalten.

11. System zum Ändern eines Wechselwirkungskomplexes (12) in einem auf einem Rechner angezeigten grafischen Modell (7) eines biochemischen Prozesses, das einer mathematischen Darstellung (6) entspricht, bei der der Wechselwirkungskomplex (12) mit einer Durchflussgeschwindigkeit (r) zwischen mindestens zwei Entitätsvariablen ($s_1$, $p_2$) in dem Prozess assoziiert wird, **gekennzeichnet durch**

Mittel zum Erhalten einer Eingabe von einem Nutzer, die eine Änderung des Wechselwirkungskomplexes **durch** Hinzufügen eines Wechselwirkungsobjektes (18) zu dem Wechselwirkungskomplex angibt, wobei das Wechselwirkungsobjekt einen ersten Endpunkt (17), der mit einer zusätzlichen Entitätsvariablen ($s_2$) assoziiert ist, und einen zweiten Endpunkt (20) hat und das Wechselwirkungsobjekt eine chemische Reaktion der zusätzlichen Entitätsvariablen ($s_2$) oder einen Einfluss der zusätzlichen Entitätsvariablen ($s_2$) auf die Durchflussgeschwindigkeit (r) darstellt,

Mittel zum grafisches Verbinden des ersten oder zweiten Endpunkts (17, 20) mit dem Wechselwirkungskomplex (12) und Anzeigen des aktualisierten Wechselwirkungskomplexes und

Mittel zum Assoziieren, in der mathematischen Darstellung (6), des aktualisierten Wechselwirkungskomplexes (12) mit einer einzelnen Durchflussgeschwindigkeit (r) in Abhängigkeit von den mindestens zwei Entitätsvariablen ($s_1$, $p_1$) und der zusätzlichen Entitätsvariablen ($s_2$).

12. System zum Ändern eines Wechselwirkungskomplexes (12) in einem auf einem Rechner angezeigten grafischen Modell (7) eines biochemischen Prozesses, das einer mathematischen Darstellung (6) entspricht, bei der der Wechselwirkungskomplex (12) mit einer Durchflussgeschwindigkeit (r) zwischen mindestens zwei Entitäten ($s_1$, $p_1$) in dem Prozess assoziiert wird,

**gekennzeichnet durch**

Mittel zum Erhalten einer Eingabe von einem Nutzer, die eine Änderung des Wechselwirkungskomplexes **durch** Entfernen eines Wechselwirkungsobjektes (14) aus dem Wechselwirkungskomplex angibt, wobei das Wechselwirkungsobjekt einen ersten Endpunkt (17), der mit einer Entitätsvariablen ($s_2$) assoziiert ist, und einen zweiten Endpunkt (20) hat und das Wechselwirkungsobjekt eine chemische Reaktion der zusätzlichen Entitätsvariablen ($s_2$) oder einen Einfluss der zusätzlichen Entitätsvariablen ($s_2$) auf die Durchflussgeschwindigkeit (r) darstellt,

Mittel zum grafischen Trennen des ersten oder zweiten Endpunkts (17, 20) von dem Wechselwirkungskomplex (12) und zum Anzeigen des aktualisierten Wechselwirkungskomplexes und

Mittel zum Assoziieren, in der mathematischen Darstellung (6), des aktualisierten Wechselwirkungskomplexes (12) mit einer einzelnen Durchflussgeschwindigkeit (r) in Abhängigkeit von den mindestens zwei übrigen verbundenen Entitäten ($s_1$, $p_1$).

## Revendications

1. Procédé de modification d'un complexe (12) d'interaction dans un modèle graphique (7) affiché par ordinateur d'un processus biochimique, ledit modèle graphique correspondant à une représentation mathématique (6) dans laquelle ledit complexe (12) d'interaction est associé à un débit (r) entre au moins deux variables d'entité ($s_1$, $p_2$) dans le processus, **caractérisé par**
une réception d'une entrée faite par un utilisateur, indiquant une modification du complexe d'interaction par addition d'un objet (18) d'interaction au complexe d'interaction,
ledit objet d'interaction comportant un premier point terminal (17) associé à une variable d'entité additionnelle ($s_2$) et un deuxième point terminal (20), ledit objet d'interaction représentant une réaction chimique de ladite variable d'entité additionnelle ($s_2$) ou une influence de ladite variable d'entité additionnelle ($s_2$) sur ledit débit (r),
une connexion graphique de l'un desdits premier et deuxième points terminaux (17, 20) avec le complexe (12) d'interaction et un affichage d'une complexe d'interaction actualisé, et
dans la représentation mathématique (6), une association du complexe (12) d'interaction actualisé avec un débit (r) unique, dépendant desdites au moins deux variables d'entité ($s_1$, $p_2$) et de ladite variable d'entité additionnelle ($s_2$).

2. Procédé de modification d'un complexe d'interaction selon la revendication 1, comprenant en outre la suppression de toute association entre le point terminal connecté (20) et une variable d'entité.

3. Procédé de modification d'un complexe d'interaction selon la revendication 1 ou 2, **caractérisé par** la délivrance à l'objet d'information connecté (18) d'une référence à l'objet (13) auquel il a été connecté, créant une connexion logique entre les objets, assurant ainsi un traitement coordonné des objets.

4. Procédé de modification d'un complexe (12) d'interaction dans un modèle graphique (7) affiché par ordinateur d'un processus biochimique, ledit modèle graphique correspondant à une représentation mathématique (6) dans laquelle ledit complexe (12) d'interaction est associé à un débit (r) entre au moins deux variables d'entité ($s_1$, $p_1$) dans le processus, **caractérisé par**
une réception d'une entrée faite par un utilisateur, indiquant une modification du complexe d'interaction par suppression d'un objet (14) d'interaction dans le complexe d'interaction,
ledit objet d'interaction comportant un premier point terminal (17) associé à une variable d'entité ($s_2$) et un deuxième point terminal (20) ledit objet d'interaction représentant une réaction chimique de ladite variable d'entité additionnelle ($s_2$) ou une influence de ladite variable d'entité additionnelle ($s_2$) sur ledit débit (r),
une déconnexion graphique de l'un des premier et deuxième points terminaux (17, 20) d'avec le complexe (12) d'interaction et un affichage d'une complexe d'interaction actualisé, et
dans la représentation mathématique (6), une association du complexe (12) d'interaction actualisé avec un débit (r) unique, dépendant des variables d'entité restantes ($s_1$, $p_1$).

5. Procédé de modification d'un complexe d'interaction selon la revendication 4, comprenant en outre la création d'une association entre le point terminal déconnecté (17) et une variable d'entité.

6. Procédé de modification d'un complexe d'interaction selon la revendication 4 ou 5, **caractérisé par** la suppression d'une référence de l'objet d'information déconnecté (18) à l'objet (13) auquel il a été connecté, supprimant toute connexion logique entre les objets.

7. Procédé de modification d'un complexe d'interaction selon la revendication 3 ou 6, dans lequel ladite connexion logique permet le commande de l'objet d'interaction connecté (18) par commande de l'objet (13) de référence auquel il a été connecté.

8. Procédé de modification d'un complexe d'interaction selon l'une quelconque des revendications précédentes, **caractérisé en ce que** ladite représentation mathématique comprend un ensemble de dérivées par rapport au temps d'entités impliquées dans le processus biochimique, chaque dérivée par rapport au temps étant définie comme une somme de contributions, où chaque contribution est proportionnelle à un débit (r).

9. Procédé de modification d'un complexe d'interaction selon la revendication 8, **caractérisé en ce que** la contribution d'un complexe d'interaction spécifique à une dérivée par rapport au temps d'une variable d'entité est proportionnelle à un coefficient stoechiométrique défini pour la variable d'entité dans ce complexe d'interaction.

10. Procédé de modification d'un complexe d'interaction selon la revendication 8 ou 9, **caractérisé par** l'actualisation automatique de l'ensemble des dérivées par rapport au temps en conformité avec le complexe d'interaction actualisé, dans le but de parvenir à une consommation et à une production relatives correctes de respectivement chaque entité de substrat et de produit.

11. Système de modification d'un complexe (12) d'interaction dans un modèle graphique (7) affiché par ordinateur d'un processus biochimique, ledit modèle graphique correspondant à une représentation mathématique (6) dans laquelle ledit complexe (12) d'interaction est associé à un débit (r) entre au moins deux variables d'entité ($s_1$, $p_2$) dans le processus, **caractérisé par**
un moyen de réception d'une entrée faite par un utilisateur, indiquant une modification du complexe d'interaction par addition d'un objet (18) d'interaction au complexe d'interaction,
ledit objet d'interaction comportant un premier point terminal (17) associé à une variable d'entité additionnelle ($s_2$) et un deuxième point terminal (20), ledit objet d'interaction représentant une réaction chimique de ladite variable d'entité additionnelle ($s_2$) ou une influence de ladite variable d'entité additionnelle ($s_2$) sur ledit débit (r),
un moyen de déconnexion graphique de l'un des premier et deuxième points terminaux (17, 20) d'avec le complexe (12) d'interaction et d'affichage d'une complexe d'interaction actualisé, et
un moyen, dans la représentation mathématique (6), d'association du complexe (12) d'interaction actualisé avec un débit (r) unique, dépendant desdites au moins deux variables d'entité ($s_1$, $p_1$) et de ladite variable d'entité additionnelle ($s_2$) .

12. Système de modification d'un complexe (12) d'interaction dans un modèle graphique (7) affiché par ordinateur d'un processus biochimique, ledit modèle graphique correspondant à une représentation mathématique (6) dans laquelle ledit complexe (12) d'interaction est associé à un débit (r) entre au moins deux variables d'entité ($s_1$, $p_1$) dans le processus, **caractérisé par**
un moyen de réception d'une entrée faite par un utilisateur, indiquant une modification du complexe d'interaction par suppression d'un objet (14) d'interaction dans le complexe d'interaction,
ledit objet d'interaction comportant un premier point terminal (17) associé à une variable d'entité ($s_2$) et un deuxième point terminal (20), ledit objet d'interaction représentant une réaction chimique de ladite variable d'entité additionnelle ($s_2$) ou une influence de ladite variable d'entité additionnelle ($s_2$) sur ledit débit (r),
un moyen de déconnexion graphique de l'un des premier et deuxième points terminaux (17, 20) d'avec le complexe (12) d'interaction et un affichage d'un complexe d'interaction actualisé, et
un moyen, dans la représentation mathématique (6), d'association du complexe (12) d'interaction actualisé avec un débit (r) unique, dépendant des variables d'entité restantes ($s_1$, $p_2$).

$$\dot{A} = -r_1 + r_4$$
$$\dot{B} = r_1 - r_2 + r_3$$
$$\dot{C} = r_2 - r_3 - r_4$$

*Fig. 1*

Fig. 2

Fig. 3A

Fig. 3B

*Fig. 3C*

| |
|---|
| Receive input |
| Connection allowed? |
| Display updated interaction complex |
| Change status of terminal points |
| Update references |
| Update flowrate |
| Update mathematical representation |

*Fig. 4*